# EUROPÄISCHE PATENTSCHRIFT

(11) **EP 2 783 647 B1**
(45) Veröffentlichungstag und Bekanntmachung des Hinweises auf die Patenterteilung: **08.11.2017**
(21) Anmeldenummer: 14161089.9
(22) Anmeldetag: 21.03.2014
(51) Int. Cl.: A61B 17/42, A61B 1/00, A61B 17/00, A61B 17/30, A61B 90/30

(54) **Colpotransilluminator zur Anordnung an einem Uterusmanipulator**
Colpotransilluminator for attachment to a uterus manipulator
Colpotransilluminateur destiné à être fixé sur un manipulateur d'utérus

(30) Priorität: 25.03.2013 DE 102013205201
(43) Veröffentlichungstag der Anmeldung: 01.10.2014
(73) Patentinhaber: Richard Wolf GmbH, 75438 Knittlingen (DE)
(72) Erfinder: Boebel, Manfred, 75245 Bauschlott (DE); Körner, Eberhard, 75438 Knittlingen (DE); Klostermann, Reiner, 75015 Bretten (DE)
(74) Vertreter: Patentanwälte Vollmann & Hemmer

(56) Entgegenhaltungen:
- EP-A1- 1 759 645
- WO-A1-2010/141423
- WO-A1-2011/140604
- WO-A2-2010/151429
- DE-A1-102009 056 705
- US-A- 5 209 754
- US-A1- 2012 330 324
- US-B1- 6 516 216

## Beschreibung

Die Erfindung betrifft einen Colpotransilluminator zur Anordnung an einem Uterusmanipulator sowie einen solchen Uterusmanipulator.

Aus DE 10 2009 056 705 ist ein Uterusmanipulator bekannt, welcher an seinem distalen Ende eine abnehmbare Doppelglocke aufweist, deren distales Ende eine Portioaufnahme, und deren proximales Ende eine Vaginaldichtung bildet. Ein solches Instrument wird für die laparoskopisch assistierte vaginale Hysterektomie verwendet und dient dazu, den Uterus von außen durch die Scheide zu halten und zu führen. Die am distalen Ende des Instrumentes angeordnete Glocke dient dabei zur Aufnahme der Portio, der Durchleuchtung des hinteren Scheidengewölbes sowie zum gasdichten Verschluss der Vagina und gegebenenfalls zusätzlich zur Isolation bei der Verwendung von HF-Instrumenten. Die Glocke soll dabei abnehmbar ausgestaltet sein und muss für die Verwendung bei verschiedenen anatomischen Gegebenheiten geeignet sein.

WO 2011/140604 offenbart einen Uterusmanipulator mit becherförmigem vaginalem Aufnahmeelement, welches zur Abdichtung gegenüber der Vagina und zur Aufnahme der Portio dient. Dieses Element hat eine konische Außenform und ist im Wesentlichen starr ausgebildet, was es erforderlich macht, verschieden dimensionierte Aufnahmeelemente für unterschiedliche anatomische Gegebenheiten vorzuhalten.

US 6,516,216 B1 offenbart einen Uterusmanipulator mit einer beleuchteten Glocke zur Aufnahme der Portio. Eine zusätzliche Vaginaldichtung ist bei diesem Instrument nicht vorgesehen.

US 5,209,254 offenbart einen Uterusmanipulator mit einer starren Portioaufnahme, welche eine ebenfalls starre Dichtscheibe aufweist, welche an ihrem Außenumfang von einem elastischen Material zur Abdichtung gegenüber der Wandung der Vagina ausgebildet ist. Auch bei diesem Instrument ist eine genaue Anpassung an verschiedene anatomische Gegebenheiten nur schwer möglich.

Es ist Aufgabe der Erfindung, einen abnehmbaren Colpotransilluminator zu schaffen, welcher zur Anordnung an einem Uterusmanipulator vorgesehen ist und eine verbesserte Anpassung an verschiedene anatomische Gegebenheiten und eine leichte Abnehmbarkeit von dem Uterusmanipulator gewährleistet.

Diese Aufgabe wird gelöst durch einen Colpotransilluminator mit den in Anspruch 1 angegebenen Merkmalen sowie durch einen Uterusmanipulator mit den in Anspruch 13 angegebenen Merkmalen.

Bevorzugte Ausführungsformen ergeben sich aus den Unteransprüchen, der nachfolgenden Beschreibung sowie den beigefügten Figuren.

Der erfindungsgemäße Colpotransilluminator ist zur Anordnung an einem Uterusmanipulator vorgesehen und ausgebildet. Dabei bildet der Colpotransilluminator dasjenige Bauteil am distalen Ende des Schaftes des Uterusmanipulators, welches zur Aufnahme der Portio und zur Abdichtung der Vagina nach außen dient. Der Colpotransilluminator stellt somit die abnehmbare Aufnahme- und Dichtglocke am distalen Ende eines Uterusmanipulators dar. Erfindungsgemäß ist der Colpotransilluminator als eine elastische rohrförmige Hülse ausgebildet, welche auf das distale Ende eines Uterusmanipulators aufgesetzt bzw. aufgezogen werden kann. Dabei bildet in Richtung der Längsachse der Hülse, welche der Längsachse des Uterusmanipulators entspricht, ein erstes distales Axialende der Hülse eine Portioaufnahme, welche bei der Verwendung des Uterusmanipulators zur Aufnahme und Fixierung der Portio, d. h. der Portio vaginalis uteri, dient. Das entgegengesetzte zweite proximale Axialende des hülsenförmigen Colpotransilluminators bildet eine Vaginaldichtung. D. h. dieser Abschnitt der Hülse kommt dichtend an der Innenwand der Vagina zur Anlage und dichtet diese so nach außen ab, so dass bei der laparoskopischen Durchtrennung des hinteren Scheidengewölbes, das im Bauchraum herrschende Pneumoteritoneum nicht aufgrund über die Scheide entweichenden Gases zusammenfällt.

Erfindungsgemäß ist die Hülse des Colpotransilluminators aus einem elastischen Material ausgebildet. Dies hat den Vorteil, dass eine gewisse elastische Verformung möglich ist, so dass eine Anpassung an verschiedene anatomische Gegebenheiten möglich ist. In einem Zentralbereich zwischen dem ersten und dem zweiten Axialende ist die Hülse des Colpotransilluminators tailliert ausgebildet. D. h. der Zentralbereich weist im Ruhezustand der Hülse einen kleineren Durchmesser als die beiden Axialenden auf. Im Bereich dieser Taillierung sind sich axial erstreckende Dehnungsfalten ausgebildet. Diese Dehnungsfalten weisen sich quer zur Umfangsrichtung der Hülse erstreckende Wandungen auf und ermöglichen, dass sich die Wandung im Zentralbereich in umfänglicher Richtung dehnen kann, so dass eine radiale Aufweitung des Innendurchmessers durch Streckung der Falten in Umfangsrichtung möglich wird. Dies ermöglicht es, den Colpotransilluminator über eine im Durchmesser erweiterte Aufnahme am distalen Ende des Uterusmanipulators zu ziehen und so den Colpotransilluminator am Distalende des Uterusmanipulators zu fixieren. Der Uterusmanipulator weist an seinem distalen Ende bevorzugt eine konische bzw. im Wesentlichen kegelstumpfförmige Aufnahme auf, wobei der größere Außendurchmesser der Aufnahme am distalen Ende und der kleinere Durchmesser weiter proximalwärts gelegen ist. Der Colpotransilluminator kann vom distalen Ende her über eine solche Aufnahme gezogen werden, so dass der kleinste Durchmesser des Colpotransilluminators im Zentralbereich proximalseitig des größten Durchmessers der Aufnahme gelegen ist. So liegt dann das erste Axialende des Colpotransilluminators, welches die Portioaufnahme bildet, distalseitig der Aufnahme am distalen Ende des Uterusmanipulators und die Vaginaldichtung liegt proximalseitig. Sowohl die Portioaufnahme als auch die Vaginaldichtung sind durch die Taillierung darüber hinaus bevorzugt im Wesentlichen glockenförmig ausgebildet.

Vorzugsweise sind mehrere Dehnungsfalten über den Umfang des Zentralbereiches verteilt angeordnet bzw. ausgebildet. Dabei sind die Dehnungsfalten weiter bevorzugt gleichmäßig über den Umfang verteilt. Besonders bevorzugt ist der gesamte Umfang mit Dehnungsfalten versehen, so dass der Zentralbereich im Querschnitt normal zur Längsachse gesehen eine über den gesamten Umfang verlaufende Zickzack- bzw. Faltenstruktur der Hülse aufweist. Die Wandungen der Falten erstrecken sich dabei im Querschnitt gesehen zickzackförmig bezüglich der Umfangsrichtung, d. h. quer bzw. im spitzen Winkel zur Umfangsrichtung. Zur Aufweitung des Zentralbereiches werden die Falten in Umfangsrichtung auseinandergezogen, so dass sich der Winkel der Wandungen der Falten zueinander vergrößert.

Gemäß einer besonders bevorzugten Ausführungsform ist in dem Zentralbereich zusätzlich zu den beschriebenen Dehnungsfalten zumindest ein sich axial erstreckender Verstärkungssteg angeordnet. Der zumindest eine Verstärkungssteg dient dazu, ein Umklappen des zweiten proximalen Axialendes, welches die Vaginaldichtung bildet, in distaler Richtung zu erschweren bzw. zu verhindern. Auch die sich quer in Umfangsrichtung erstreckenden Wandungen der Falten führen zu einem Verstärkungseffekt, welcher zusätzlich das Umklappen verhindert. Bevorzugt sind mehrere Verstärkungsstege über den Umfang verteilt angeordnet. Insbesondere sind mehrere gleichmäßig über den Umfang verteilte Verstärkungsstege vorgesehen, beispielsweise vier Verstärkungsstege, welche im Winkel von jeweils 90° zueinander angeordnet sind. Der zumindest eine bzw. die mehreren Verstärkungsstege sind vorzugsweise am Außenumfang des Zentralbereiches angeordnet. Bevorzugt schneiden dabei die Verstärkungsstege bzw. schneidet der zumindest eine Verstärkungssteg die Taillierung sehnenförmig, d. h. der Verstärkungssteg bildet eine sich quer zum Umfang erstreckende Wandung, welche die beiden einander zugewandten konischen Außenflächen der Taillierung sehnenförmig verbindet.

Der zumindest eine Verstärkungssteg steht dabei bevorzugt in radialer Richtung vom Außenumfang des Zentralbereiches vor. Der Innenumfang des Zentralbereiches ist durch den oder die Verstärkungsstege dabei vorteilhafterweise nicht beeinflusst, so dass der Innenumfang des Zentralbereiches bevorzugt vollumfänglich an einer Aufnahme des Uterusmanipulators zur Anlage kommen kann.

Weiter bevorzugt erstreckt sich der zumindest eine Verstärkungssteg in axialer Richtung parallel zu den Dehnungsfalten, welche sich ebenfalls in axialer Richtung vom proximalen zum distalen Ende, d. h. parallel zur Instrumentenlängsachse erstrecken. So wird eine optimale Verstärkung, welche ein Umklappen des zweiten Axialendes der Hülse verhindert, erreicht.

Weiter bevorzugt weist der Colpotransilluminator zumindest im Bereich eines Axialendes, vorzugsweise zumindest im Bereich beider Axialenden einen kreisförmigen Querschnitt auf. Dabei weist die Hülse, welche den Colpotransilluminator bildet, in ihrer Ruhelage (d. h. ohne elastische Verformung) den entsprechenden kreisförmigen Querschnitt auf, durch elastische Verformung kann die Form verändert werden. Besonders bevorzugt weist der Colpotransilluminator über seine gesamte axiale Länge einen Querschnitt von kreisförmiger Grundform auf. D. h. in dem Zentralbereich, in welchem die Dehnungsfalten angeordnet sind, sind diese entlang einer kreisförmigen Umfangslinie verteilt angeordnet.

Gemäß einer weiteren bevorzugten Ausführungsform sind die Durchmesser der beiden Axialenden des Colpotransilluminators unterschiedlich groß ausgebildet, wobei vorzugsweise das die Vaginaldichtung bildende zweite Axialende einen größeren Durchmesser aufweist als das die Portioaufnahme bildende erste Axialende. In dem zwischenliegenden taillierten Zentralbereich ist der Durchmesser, insbesondere der Außendurchmesser dabei vorzugsweise kleiner als der Außendurchmesser an dem ersten Axialende, welches die Portioaufnahme ausbildet. Durch die größere Ausgestaltung des Durchmessers der Vaginaldichtung wird eine zuverlässige Abdichtung an der Innenwand der Vagina erreicht.

Der Colpotransilluminator weist weiter bevorzugt an zumindest einem Axialende, vorzugsweise an beiden Axialenden einen wulstartigen Ring auf. D. h. an den Axialenden ist die Hülse bevorzugt verdickt ausgebildet. Dieser verdickte wulstartige Ring gibt dem Axialende eine gewisse Formstabilität, welche dazu beiträgt, das Bauteil in seiner Ruhelage in die vorzugsweise kreisförmige Grundform zu bewegen.

Gemäß einer weiteren bevorzugten Ausführungsform weist der Colpotransilluminator an seinem ersten, die Portioaufnahme bildenden Axialende einen nach innen gerichteten Kragen auf, welcher zum Umgreifen eines distalen Endabschnittes des Uterusmanipulators ausgebildet ist. So kann das erste Axialende des Colpotransilluminators dann die distale vordere Stirnkante des Uterusmanipulators bilden. So bildet der Kragen am distalen Ende des Uterusmanipulators, wenn der Colpotransilluminator auf diesem aufgesetzt ist, eine elastische Innenwandung für die Portioaufnahme, welche zu einer guten und dichten Anlage am Gewebe führt.

Der Kragen erstreckt sich vorzugsweise zumindest anteilig in axialer Richtung proximalwärts. D. h. er ist am distalen Ende des Colpotransilluminators nicht oder nicht nur radial nach innen gerichtet, sondern erstreckt sich bevorzugt im spitzen Winkel zur Instrumentenlängsachse bzw. Längsachse des Colpotransilluminators oder parallel zur Längsachse in Richtung des proximalen Endes. Der Kragen kann so eine glockenförmige Aufnahme am distalen Ende des Uterusmanipulators am Innenumfang umgreifen. Der Colpotransilluminator erstreckt sich dabei um die distale Stirnkante dieser glockenförmigen Aufnahme und dann über deren Außenumfang weiter proximalwärts zu seinem zweiten Proximalende. So wird die distale Stirnkante des Uterusmanipulators vollständig von dem Colpotransilluminator umschlossen, was der sicheren Portioaufnahme, einer verbesserten Beleuchtung und elektrischen Isolierung bei Verwendung von HF-Instrumenten dient.

Um die gewünschte Beleuchtungsfunktion übernehmen zu können, ist der Colpotransilluminator vorteilhafterweise zumindest an seinem ersten Axialende und vorzugsweise vollständig aus einem transparenten Material gefertigt. In dem Uterusmanipulator sind am distalen Ende Beleuchtungselemente, wie beispielsweise Leuchtdioden oder die Enden von Lichtleitfasern angeordnet. Das von diesen emittierte Licht kann das transparente Material des Colpotransilluminators an dessen ersten distalen Axialende durchdringen, wobei das erste axiale Ende des Colpotransilluminators dabei bevorzugt so ausgestaltet ist, dass es für eine Lichtverteilung und Abstrahlung bevorzugt in distaler Richtung sorgt. Dies kann beispielsweise über eine wulstförmige Verdickung am distalen Ende erfolgen. Das so abgestrahlte Licht dient bei der Operation der Durchleuchtung des hinteren Scheidengewölbes. Bevorzugt ist der gesamte Colpotransilluminator aus transparentem Material gefertigt. Dies ergibt sich insbesondere dann, wenn der Colpotransilluminator, wie weiter bevorzugt, einstückig aus einem Kunststoffmaterial, wie beispielsweise Silikon, gefertigt ist.

Gemäß einer weiteren bevorzugten Ausgestaltung ist der Zentralbereich mit den Dehnungsfalten derart ausgestaltet, dass sich der Zentralbereich im Bereich seines kleinsten Durchmessers durch die Dehnungsfalten elastisch auf zumindest einem Durchmesser dehnen kann, welcher dem Innendurchmesser an dem ersten, d. h. dem distalen Axialende entspricht. Diese Ausgestaltung ermöglicht es, dass ein Uterusmanipulator mit seiner am distalen Ende ausgebildeten Aufnahme von dem zweiten proximalen Ende her in den Colpotransilluminator eingeführt werden kann, wobei die Aufnahme dann unter Erweiterung des Zentralbereiches diesen passieren kann und im Bereich des distalen Endes des Colpotransilluminators an dessen Innenumfang zur Anlage kommt. Dabei wird das distale Ende der Aufnahme des Uterusmanipulators bevorzugt, wie vorangehend beschrieben, von einem Kragen am distalen Ende des Colpotransilluminators umgriffen. Der Uterusmanipulator weist bevorzugt eine starre Aufnahme für den Colpotransilluminator auf. Durch die Dehnbarkeit aufgrund der Dehnungsfalten im Zentralbereich ist es dennoch möglich, dass diese starre Aufnahme durch den verengten bzw. taillierten Zentralbereich unter dessen elastischer Aufweitung hindurchgeführt werden kann.

Gegenstand der Erfindung ist ferner ein Uterusmanipulator, insbesondere für die laparoskopisch assistierte vaginale Hysterektomie, welcher einen Schaft aufweist, an welchem ein distaler Endabschnitt und eine am proximalen Ende gelegene Handhabe angeordnet ist. Der distale Endabschnitt bildet eine Aufnahme, auf welche ein Colpotransilluminator gemäß der vorangehenden Beschreibung lösbar aufgesetzt ist. Der Colpotransilluminator stellt eine Aufnahme- und Dichtglocke dar. Der Colpotransilluminator bildet dabei mit seinem distalen Ende eine Portioaufnahme und mit seinem proximalen Ende eine Vaginaldichtung, wie es vorangehend beschrieben wurde. Bevorzugt weist der Uterusmanipulator an seinem distalen Endabschnitt, welcher die Aufnahme für den Colpotransilluminator bildet, eine radiale Aufweitung auf, auf welche der Colpotransilluminator lösbar aufgesetzt ist. Insbesondere ist das distale Ende des Uterusmanipulators im Wesentlichen glockenförmig ausgebildet, wobei sich der die Aufnahme bildende Endabschnitt zum distalen Ende hin radial aufweitet, so dass er eine im Wesentlichen konische bzw. kegelstumpfförmige Außenkontur aufweist, auf welcher der Colpotransilluminator mit seiner distalseitig des engsten Abschnittes gelegenen Innenkontur anliegt. Aufgrund der Taillierung des Colpotransilluminators ist es dabei möglich, dass dieser mit seiner Innenumfangsfläche am konischen bzw. kegelstumpfförmigen Bereich der Aufnahme des Uterusmanipulators anliegt. Im Zentralbereich des Colpotransilluminators kommen dabei die Dehnungsfalten mit ihren radial innengelegenen Abschnitten an dieser Außenumfangsfläche des Uterusmanipulators zur Anlage.

D. h. bevorzugt ist der Uterusmanipulator in seinem distalen Endabschnitt so ausgebildet, dass er sich von einer Aufweitung am distalen Endabschnitt in proximaler Richtung verjüngt, wobei diese Verjüngung in ihrer Form eine im Wesentlichen konische Außenkontur aufweist, welche der konischen Innenkontur der distalen Seite des taillierten Zentralbereiches des Colpotransilluminators derart angepasst ist, dass dieser mit seinem Innenumfang an der Verjüngung des Uterusmanipulators zur Anlage kommen kann. Hinsichtlich der weiteren Ausgestaltung des Uterusmanipulators wird auf die vorangehende Beschreibung des Colpotransilluminators verwiesen, in deren Zusammenhang die wesentlichen Aspekte des Uterusmanipulators bereits beschrieben wurden.

Nachfolgend wird die Erfindung beispielhaft anhand der beigefügten Figuren beschrieben. In diesen zeigt:

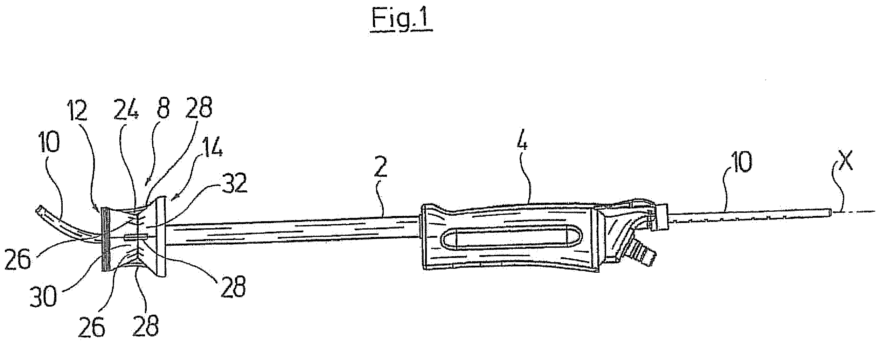

- Fig. 1: schematisch einen Uterusmanipulator mit dem erfindungsgemäßen Colpotransilluminator,
- Fig. 2: eine Seitenansicht des Colpotransilluminators gemäß Fig. 1,
- Fig. 3: eine stirnseitige Draufsicht auf den Colpotransilluminator gemäß Fig. 2,
- Fig. 4: eine Schnittansicht des Instrumentes gemäß Fig. 1, und
- Fig. 5: vergrößert das distale Ende des Uterusmanipulators gemäß Fig. 4.

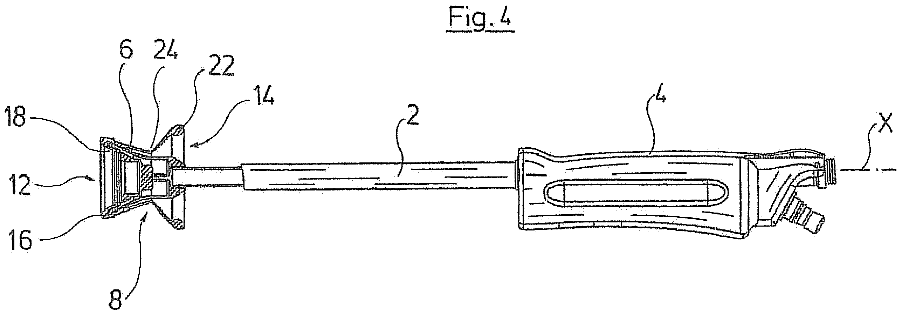

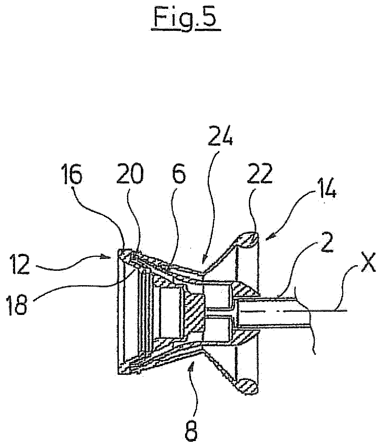

Der erfindungsgemäße Uterusmanipulator weist einen Schaft 2 auf, an dessen proximalen Ende eine Handhabe 4 und an dessen distalen Ende ein distaler Endabschnitt gebildet ist, welcher als eine Aufnahme 6 (siehe Figuren 4 und 5) für den Colpotransilluminator 8 ausgebildet ist. Dieser stellt eine Aufnahme- und Dichtglocke dar und ist auf die Aufnahme 6 aufgesetzt. Durch das Innere des Schaftes 2 ist eine Hohlsonde 10 geführt, welche am distalen Ende des Schaftes 2 aus dem Colpotransilluminator 8 austritt und in distaler Richtung vorsteht. Die Hohlsonde 10 ist im Schaft 2 in axialer Richtung X in definierten Rastschritten verschiebbar.

Der Colpotransilluminator 8 bildet an seinem ersten distalen Axialende 12 eine Portioaufnahme und an seinem entgegengesetzten zweiten proximalen Axialende 14 eine Vaginaldichtung. Der Colpotransilluminator 8 ist aus einem elastischen transparenten Material, insbesondere einem elastischen Kunststoff bzw. Elastomer wie beispielsweise Silikon, ausgebildet.

An seinem ersten Axialende 12 weist der hülsenförmige Colpotransilluminator 8 einen kreisringförmigen umlaufenden Wulst 16 auf. Von diesem ausgehend erstreckt sich am Innenumfang ein nach innen und zum proximalen Ende in Richtung der Längsachse X gerichteter Kragen 18. Der Kragen 18 erstreckt sich über den gesamten Innenumfang des distalen Endes 12 des Colpotransilluminators 8 im spitzen Winkel zur Längsachse X proximalwärts. Der Kragen 18 umgreift so das distale Ende der Aufnahme 6 am distalen Ende des Schaftes 2 des Uterusmanipulators. Die Aufnahme 6 ist an ihrem distalen Ende radial aufgeweitet, d. h. weist dort den größten Durchmesser auf und verjüngt sich in proximaler Richtung, so dass insgesamt eine konische bzw. kegelstumpfförmige Form am Außenumfang gebildet wird. Am Innenumfang ist eine ähnliche Kontur gegeben, so dass das distale Ende der Aufnahme 6 glockenförmig ausgebildet ist. Der distale ringförmige Rand 20 der Aufnahme 6 wird von dem Kragen 18 übergriffen, so dass der Wulst 16 axialseitig des Randes 20 gelegen ist. Die Aufnahme 6 mit dem übergestülpten Kragen 18 bildet die Portioaufnahme, mit welcher bei der Operation die Portio fixiert wird. Im Bereich des Randes 20 sind Lichtaustrittsöffnungen von Lichtleitern (hier nicht gezeigt) oder Beleuchtungselemente wie Leuchtdioden angeordnet, welche ihr emittiertes Licht in den Wulst 16 einkoppeln, so dass das Licht aus diesen nach außen, insbesondere in distaler Richtung strahlt und so bei der Operation das hintere Scheidengewölbe durchleuchtet.

Der Colpotransilluminator 8 weist an seinem zweiten proximalen Axialende 14 einen ringförmigen Wulst 22 auf, welcher eine Kreisringform hat, jedoch elastisch verformbar ist. Der Wulst 22 sorgt aufgrund seiner elastischen Rückstellkräfte dafür, dass das zweite Axialende 14 in seiner Ruhelage in dieser Form gehalten wird. Das zweite proximale Axialende 14 mit dem Wulst 22 bildet eine Vaginaldichtung, welche an der Innenwand der Vagina dichtend zur Anlage kommen kann. Um sich hier der gegebenen anatomischen Form anpassen zu können, ist der hülsenförmige Colpotransilluminator insgesamt und auch im Bereich des Wulstes 22 elastisch verformbar. Durch die elastischen Rückstellkräfte wird er dabei in dichter Anlage mit der Innenwand der Vagina gehalten. Das proximale Ende des Colpotransilluminators 8 bildet somit ebenfalls eine Glocke, welche zum proximalen Ende hin geöffnet ist, d. h. entgegengesetzt zu der von dem ersten Axialende 12 gebildeten Glocke, welche die Portioaufnahme bildet.

In einem Zentralbereich zwischen dem ersten distalen Axialende 12 und dem zweiten proximalen Axialende 14 ist der hülsenförmige Colpotransilluminator tailliert ausgebildet. D. h. hier gibt es eine radial nach innen gerichtete Einschnürung 24. Die Einschnürung 24 definiert den kleinsten Durchmesser des Colpotransilluminators. An seinem zweiten Axialende 14, welches die Vaginaldichtung bildet, weist der Colpotransilluminator 8 seinen größten Außendurchmesser auf. Am ersten Axialende 12, welches die Portioaufnahme bildet, weist der Colpotransilluminator einen Außendurchmesser auf, welcher größer als der Außendurchmesser im Bereich der Einschnürung 24 ist, in diesem Beispiel jedoch kleiner als der Außendurchmesser des Wulstes 22 am zweiten Axialende 14 ist. Im Bereich der Einschnürung 24 ist im entspannten Zustand des Materials des Colpotransilluminators der Innendurchmesser kleiner als der Außendurchmesser des Randes 20 der Aufnahme 6 des Uterusmanipulators. Um den Colpotransilluminator 8 vom distalen Ende her über die Aufnahme 6 ziehen zu können, ist es erforderlich, die Aufnahme 6 mit dem Rand 20 voran in das zweite Axialende 14 des Colpotransilluminators 8 einzuführen. Anschließend muss die Einschnürung 24 in Umfangsrichtung bzw. radialer Richtung so aufgeweitet werden, dass der Rand 20 der Aufnahme 6 die Einschnürung 24 passieren kann.

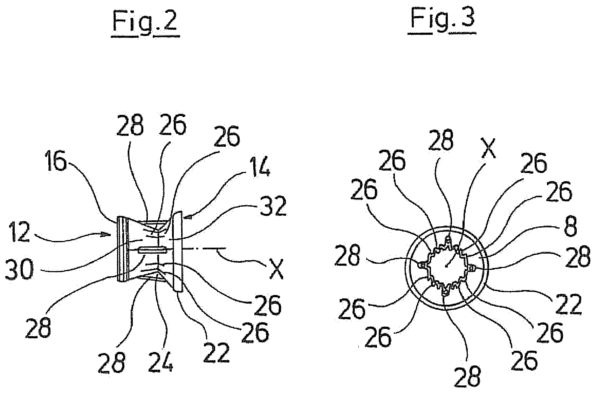

Dies wird erfindungsgemäß durch die Anordnung von Dehnungsfalten 26 begünstigt. Die Dehnungsfalten 26 sind in dem Zentralbereich im Bereich der Einschnürung 24 angeordnet und erstrecken sich zur Längsachse X. Wie in Fig. 3 zu erkennen ist, bilden die Dehnungsfalten 26 in einen Querschnitt quer zur Längsachse X in Umfangsrichtung einen zickzackförmigen Verlauf der Wandung des Colpotransilluminators 8. Die Dehnungsfalten 26 ermöglichen eine starke Dehnung in Umfangsrichtung, so dass der Rand 20 der Aufnahme 6 die Einschnürung 24 ohne Probleme unter elastischer Aufweitung des Colpotransilluminators 8 passieren kann. Wenn der Colpotransilluminator 8 an der Aufnahme 6 anliegt, liegt der distalseitige Bereich der Taillierung, d. h. der konische bzw. kegelstumpfförmige Abschnitt des Colpotransilluminators 8, welcher distalseitig der Einschnürung 24 gelegen ist, an der Außenseite bzw. am Außenumfang der Aufnahme 6 an. In diesem Bereich liegen dann die Innenkanten der Dehnungsfalten 26, welche eine distale konische Fläche 30 definieren, auf der Außenfläche der Aufnahme 6 an. So wird eine feste Anlage des Colpotransilluminators 8 an der Aufnahme 6 gewährleistet. Darüber hinaus wird im Bereich der Einschnürung 24 ein möglichst kleiner Außendurchmesser geschaffen, so dass der Raum, welchen das Instrument in der Scheide einnimmt, minimiert wird und so ein ausreichender Freiraum zur Operation gelassen wird.

Um bei Bewegung des Uterusmanipulators mit dem Colpotransilluminator 8 in proximaler Richtung in der Vagina ein Umklappen des proximalen Abschnittes des Colpotransilluminators 8 und des Wulstes 22 zu verhindern, sind an dem hier gezeigten Colpotransilluminator darüber hinaus Verstärkungsstege 28 ausgebildet. Die Verstärkungsstege 28 erstrecken sich in radialer Richtung rippenförmig am Außenumfang des Colpotransilluminators 8 nach außen. Dabei erstrecken sich die Verstärkungsstege 28 parallel zu den Dehnungsfalten 26 und zur Instrumentenlängsachse X. Im Bereich der Taillierung erstrecken sich die Verstärkungsstege 28 so, dass sie sich sehnenförmig über die Einschnürung 24 hinweg erstrecken und so die distale konische Fläche 30 mit der proximalen konischen Fläche 32 angrenzend an die Einschnürung 24 verbinden. Im hier gezeigten Beispiel sind vier Verstärkungsstege 28 vorgesehen, welche jeweils im Winkel von 90° zueinander angeordnet sind. Direkt angrenzend an die Verstärkungsstege 28 sind Dehnungsfalten 26 angeordnet, so dass die Verstärkungsstege 28 die Dehnbarkeit im Bereich der Einschnürung 24, d. h. im Zentralbereich des hülsenförmigen Colpotransilluminators 8 im Wesentlichen nicht einschränken. Die Verstärkungsstege 28 stützen insbesondere die proximale konische Fläche 32 bzw. konische Außenkontur, welche die Einschnürung 24 mit dem Wulst 22 am zweiten Axialende 14 verbindet, so dass diese proximale konische Fläche 32 gegen ein unbeabsichtigtes Umklappen in distaler Richtung gesichert wird. So wird beim Zurückziehen des Colpotransilluminators in proximaler Richtung in der Vagina ein Umklappen der Vaginaldichtung verhindert.

Der gesamte Colpotransilluminator 8 ist in diesem Beispiel einstückig aus Kunststoff, insbesondere aus Elastomer oder Silikon gefertigt und transparent ausgebildet, um eine lichtleitende Funktion im Bereich des distalen Axialendes 12 zu bewirken.

### Bezugszeichenliste

- 2: - Schaft
- 4: - Handhabe
- 6: - Aufnahme
- 8: - Colpotransilluminator
- 10: - Hohlsonde
- 12: - erstes Axialende, Portioaufnahme
- 14: - zweites Axialende, Vaginaldichtung
- 16: - Wulst
- 18: - Kragen
- 20: - Rand
- 22: - Wulst
- 24: - Einschnürung
- 26: - Dehnungsfalten
- 28: - Verstärkungsstege
- 30: - distale konische Fläche
- 32: - proximale konische Fläche
- X: - Instrumentenlängsachse

## Patentansprüche

1. Colpotransilluminator (8) zur Anordnung an einem Uterusmanipulator, wobei der Colpotransilluminator (8)
hülsenförmig aus einem elastischen Material ausgebildet ist,
an einem ersten distalen Axialende (12) eine Portioaufnahme und an einem zweiten proximalen Axialende (14) eine Vaginaldichtung bildet, in einem Zentralbereich (24) zwischen ersten (12) und zweiten (14) Axialende tailliert ausgebildet ist, und
im Bereich der Taillierung sich axial erstreckende Dehnungsfalten (26) aufweist.

2. Colpotransilluminator nach Anspruch 1, bei welchem in dem Zentralbereich (24) zumindest ein sich axial erstreckender Verstärkungssteg (28) angeordnet ist.

3. Colpotransilluminator nach Anspruch 2, bei welchem der zumindest eine und vorzugsweise mehrere Verstärkungsstege (28) am Außenumfang des Zentralbereiches (24) angeordnet sind.

4. Colpotransilluminator nach Anspruch 2 oder 3, bei welchem der zumindest eine Verstärkungssteg (28) in radialer Richtung vom Außenumfang des Zentralbereiches (24) vorsteht.

5. Colpotransilluminator nach einem der Ansprüche 2 bis 4, bei welchem sich der zumindest eine Verstärkungssteg (28) in axialer Richtung parallel zu den Dehnungsfalten (26) erstreckt.

6. Colpotransilluminator nach einem der vorangehenden Ansprüche, welcher zumindest im Bereich eines Axialendes (12, 14) und vorzugsweise zumindest im Bereich beider Axialenden (12, 14) einen kreisförmigen Querschnitt aufweist.

7. Colpotransilluminator nach einem der vorangehenden Ansprüche, bei welchem die Durchmesser der Axialenden (12, 14) unterschiedlich groß sind, wobei vorzugsweise das die Vaginaldichtung bildende zweite Axialende (14) einen größeren Durchmesser aufweist als das die Portioaufnahme bildende erste Axialende (12).

8. Colpotransilluminator nach einem der vorangehenden Ansprüche, wobei der Colpotransilluminator (8) an zumindest einem Axialende (12, 14), vorzugsweise an beiden Axialenden (12, 14) einen wulstartigen Ring (16, 22) aufweist.

9. Colpotransilluminator nach einem der vorangehenden Ansprüche, welcher an seinem ersten die Portioaufnahme bildenden Axialende einen nach innen gerichteten Kragen (18) aufweist, welcher zum Umgreifen eines distalen Endabschnittes (6) des Uterusmanipulators ausgebildet ist.

10. Colpotransilluminator nach Anspruch 9, bei welchem sich der Kragen (18) zumindest anteilig in axialer Richtung zum proximalen Ende (14) hin erstreckt.

11. Colpotransilluminator nach einem der vorangehenden Ansprüche, wobei der Colpotransilluminator zumindest an seinem ersten Axialende (12) und bevorzugt vollständig aus einem transparenten Material besteht.

12. Colpotransilluminator nach einem der vorangehenden Ansprüche, bei welchem der Zentralbereich (24) mit den Dehnungsfalten (26) derart ausgestaltet ist, dass sich der Zentralbereich (24) im Bereich seines kleinsten Durchmessers durch die Dehnungsfalten (26) elastisch auf zumindest einen Durchmesser dehnen kann, welcher dem Innendurchmesser an dem ersten Axialende (12) entspricht.

13. Uterusmanipulator, insbesondere für die laparoskopisch assistierte vaginale Hysterektomie, mit einem Schaft (2), an welchem ein distaler Endabschnitt und eine proximale Handhabe angeordnet sind, wobei auf dem distalen Endabschnitt (6) ein Colpotransilluminator (8) nach einem der vorangehenden Ansprüche lösbar aufgesetzt ist.

14. Uterusmanipulator nach Anspruch 13, welcher an seinem distalen Endabschnitt (6) eine radiale Aufweitung (20) aufweist, auf welcher der Colpotransilluminator (8) lösbar aufgesetzt ist.

15. Uterusmanipulator nach Anspruch 14, welcher sich ausgehend von der Aufweitung (20) am distalen Endabschnitt (6) in proximaler Richtung verjüngt, wobei diese Verjüngung in ihrer Form derart an eine konische Innenkontur der distalen Seite (30) des taillierten Zentralbereiches des Colpotransilluminators (8) angepasst ist, dass dieser mit seinem Innenumfang an der Verjüngung anliegt.

## Claims

1. A colpotransilluminator (8) for the arrangement on an uterus manipulator, wherein the colpotransilluminator (8)
is designed in a sleeve-like manner of an elastic material,
at a first distal axial end (12) forms a portio receiver and at a second proximal axial end (14) forms a vaginal seal,
is designed in a waisted manner in a central region (24) between the first (12) and the second (14) axial end,
and comprises axially extending stretch folds (26) in the region of the waist.

2. A colpotransilluminator according to claim 1, with which at least one axially extending reinforcement web (28) is arranged in the central region (24).

3. A colpotransilluminator according to claim 2, with which the at least one and preferably several reinforcement webs (28) are arranged on the outer periphery of the central region (24).

4. A colpotransilluminator according to claim 2 or 3, with which the at least one reinforcement web (28) projects in the radial direction from the outer periphery of the central region (24).

5. A colpotransilluminator according to one of the claims 2 to 4, with which the at least one reinforcement web (28) extends in the axial direction parallel to the stretch folds (26).

6. A colpotransilluminator according to one of the preceding claims, which at least in the region of an axial end (12, 14) and preferably at least in the region of both axial ends (12, 14) has a circular cross section.

7. A colpotransilluminator according to one of the preceding claims, with which the diameter of the axial ends (12, 14) are differently large, wherein preferably the second axial end (14) forming the vaginal seal has a greater diameter than the first axial end (12) forming the portio receiver.

8. A colpotransilluminator according to one of the preceding claims, wherein the colpotransilluminator (8) comprises a bead-like ring (16, 22) at least at one axial end (12, 14) preferably at both axial ends (12, 14).

9. A colpotransilluminator according to one of the preceding claims, which at its first axial end forming the portio receiver comprises an inwardly directed collar (18) which is designed for encompassing a distal end section (6) of the uterus manipulator.

10. A colpotransilluminator according to claim 9, with which the collar (18) at least in parts extends in the axial direction to the proximal end (14).

11. A colpotransilluminator according to one of the preceding claims, wherein the colpotransilluminator consists of a transparent material at least at its first axial end (12) and preferably completely.

12. A colpotransilluminator according to one of the preceding claims, with which the central region (24) with the stretch folds (26) is designed in a manner such that the central region (24) in the region of its smallest diameter, by way of the stretch folds (26) can stretch elastically to at least a diameter which corresponds to the inner diameter at the first axial end (12).

13. An uterus manipulator, in particular for laparasopically assisted vaginal hysterectomy, with a shank (2), on which a distal end section and a proximal handle are arranged, wherein a colpotransilluminator (8) according to one of the preceding claims is releasably applied on the distal end section (6).

14. An uterus manipulator according to claim 13, which at its distal end section (6) comprises a radial widening (20), on which the colpotransilluminator (8) is releasably applied.

15. An uterus manipulator according to claim 14, which departing from the widening (20) at the distal end section (6) tapers in the proximal direction, wherein this tapering in its shape is adapted to a conical inner contour of the distal side (30) of the waisted central region of the colpotransilluminator (8) in a manner such that this colpotransilluminator bears with its inner periphery on the tapering.

## Revendications

1. Colpotransilluminateur (8) à disposer au niveau d'un manipulateur utérin, dans lequel le colpotransilluminateur (8) est exécuté en forme de gaine dans un matériau élastique, forme au niveau d'une première extrémité axiale distale (12) une réception de portion vaginale du col de l'utérus, et au niveau d'une seconde extrémité axiale proximale (14) une étanchéité vaginale, est exécutée de façon cintrée dans une zone centrale (24) entre la première (12) et la seconde (14) extrémités axiales, et présente dans la zone du cintrage des plis d'expansion (26) qui s'étendent dans la direction axiale.

2. Colpotransilluminateur selon la revendication 1, dans lequel est disposée dans la zone centrale (24) au moins une nervure de renforcement (28) qui s'étend au niveau axial.

3. Colpotransilluminateur selon la revendication 2, dans lequel les nervures de renforcement (28), au moins au nombre de une et de préférence de plusieurs, sont disposées au niveau de la périphérie extérieure de la zone centrale (24).

4. Colpotransilluminateur selon la revendication 2 ou 3, dans lequel la nervure de renforcement (28), au moins au nombre de une, dépasse dans la direction radiale de la périphérie extérieure de la zone centrale (24).

5. Colpotransilluminateur selon l'une des revendications 2 à 4, dans lequel la nervure de renforcement (28), au moins au nombre de une, s'étend dans la direction axiale parallèlement aux plis d'expansion (26).

6. Colpotransilluminateur selon l'une des revendications précédentes, qui présente une section circulaire au moins dans la zone d'une extrémité axiale (12, 14), et de préférence au moins dans la zone des deux extrémités axiales (12, 14).

7. Colpotransilluminateur selon l'une des revendications précédentes, dans lequel les diamètres des extrémités axiales (12, 14) sont de taille différente, où de préférence la seconde extrémité axiale (14) formant l'étanchéité vaginale présente un diamètre supérieur à la première extrémité axiale (12) formant la réception de portion vaginale du col.

8. Colpotransilluminateur selon l'une des revendications précédentes, dans lequel le colpotransilluminateur (8) présente au niveau d'au moins une extrémité axiale (12, 14), de préférence au niveau des deux extrémités axiales (12, 14), un anneau de type boudin (16,22).

9. Colpotransilluminateur selon l'une des revendications précédentes, qui présente au niveau de sa première extrémité axiale formant la réception de portion vaginale du col une collerette (18) orientée vers l'intérieur qui est exécutée pour entourer un segment d'extrémité distal (6) du manipulateur utérin.

10. Colpotransilluminateur selon la revendication 9, dans lequel la collerette (18) s'étend au moins partiellement dans la direction axiale vers l'extrémité proximale (14).

11. Colpotransilluminateur selon l'une des revendications précédentes, dans lequel le colpotransilluminateur se compose au moins au niveau de sa première extrémité axiale (12), et de préférence entièrement, d'un matériau transparent.

12. Colpotransilluminateur selon l'une des revendications précédentes, dans lequel la partie centrale (24) présentant les plis d'expansion (26) est exécutée de façon telle que la zone centrale (24) peut, dans la zone de son plus petit diamètre, s'étendre de façon élastique, via les plis d'expansion (26), à au moins un diamètre qui correspond au diamètre intérieur au niveau de la première extrémité axiale (12).

13. Manipulateur utérin, en particulier pour hystérectomie vaginale assistée par laparoscopie, comportant une tige (2) au niveau de laquelle sont disposés un segment d'extrémité distal et une zone de manipulation proximale, où au niveau du segment d'extrémité distal (6) est fixé de façon amovible un colpotransilluminateur (8) selon l'une des revendications précédentes.

14. Manipulateur utérin selon la revendication 13, qui présente au niveau de son segment d'extrémité distal (6) un élargissement radial (20) sur lequel est fixé de façon amovible le colpotransilluminateur (8).

15. Manipulateur utérin selon la revendication 14, qui, en partant de l'élargissement (20), se rétrécit au niveau du segment d'extrémité distal (6) dans la direction proximale, ce rétrécissement, dans sa forme, étant adapté, au niveau d'un contour intérieur conique, au côté distal (30) de la zone centrale cintrée du colpotransilluminateur (8), de façon telle que celui-ci repose par sa périphérie intérieure au niveau du rétrécissement.
